# EUROPEAN PATENT APPLICATION

(11) **EP 2 664 266 A1**
(43) Date of publication of application: **20.11.2013**
(21) Application number: 12168212.4
(22) Date of filing: 16.05.2012
(51) Int. Cl.: A61B 1/00, A61B 1/04

(54) **Medical inspection device**

(71) Applicant: Apple Biomedical Inc., New Taipei City 24159 (TW)
(72) Inventor: Huang, Tzai-Kun, 24159 New Taipei City (TW)
(74) Representative: Viering, Jentschura & Partner

(57) **Abstract**

A medical inspection device which comprises a light-transmitting unit, a light-capturing unit detachably coupled to the light-transmitting unit and a hand-held display unit detachably coupled to the light-capturing unit. The light-transmitting unit comprises at least a first optical lens for receiving and transmitting light. The light-capturing unit comprises a light sensitive element and a signal output end connected to the light sensitive element, wherein the light sensitive element is configured to receive the light via the light-transmitting unit and to generate an electrical signal based on the received light. The hand-held display unit is configured to receive the electrical signal from the signal output end, wherein the hand-held display unit comprises a hand-held portion and a display portion for displaying an image based on the received electrical signal.

## Description

### FIELD OF THE INVENTION

The present invention generally relates to a medical inspection device. More particularly, the present invention relates to a medical inspection device adaptable for various medical inspection functions. The medical inspection device comprises a light transmitting unit, a light capturing unit detachably coupled to the light transmitting unit, and a hand-held display unit detachably coupled to the light capturing unit.

### BACKGROUND OF THE INVENTION

There is a trend of providing medical inspection devices which may be used for examining a variety of organs, such as the eye, ear, skin or internal organs, and producing images thereof. WO 2009/004115 discloses a medical inspection device comprising an optical component connectable to a camera unit. The optical component comprises a data structure including data associated with the optical component. When the optical component is connected to the camera unit, data associated with the optical component is transferred to the camera unit. Image production of an organ by the camera unit is controlled based on the data associated with the optical component. The camera unit comprises a display and an image sensor for producing image of an organ.

The aforesaid device may be complicated in design because each optical component needs to include a data structure such that the images produced by the optical component may be transferred to the camera unit. Furthermore, the image sensor is integrated with the camera unit, which limits the selection of the image sensor. In particular, the image resolutions required for examining different organs are different. The aforesaid device thus may require a high-resolution image sensor to meet certain examination purposes. During certain medical examinations, it is required to operate several medical inspection devices, where each medical inspection device is equipped with an image sensor of a particular resolution. The cost associate with which is therefore higher.

In view of this, it is desirable to provide an improved medical inspection device that addresses the shortcomings of the prior art devices.

### BRIEF SUMMARY OF THE INVENTION

Examples of the present invention may provide a medical inspection device which comprises a light-transmitting unit, a light-capturing unit detachably coupled to the light-transmitting unit and a hand-held display unit detachably coupled to the light-capturing unit. The light-transmitting unit comprises at least a first optical lens for receiving and transmitting light. The light-capturing unit comprises a light sensitive element and a signal output end connected to the light sensitive element, wherein the light sensitive element is configured to receive the light via the light-transmitting unit and to generate an electrical signal based on the received light. The hand-held display unit is configured to receive the electrical signal from the signal output end. The hand-held display unit comprises a hand-held portion and a display portion for displaying an image based on the received electrical signal.

Other examples of the present invention may provide a medical inspection device which comprises an inspection unit, a light-capturing unit, and a hand-held display unit. The inspection unit has a front end with a light transmitting unit disposed therein and a rear end. The light-capturing unit is detachably coupled to the rear end of the inspection unit, and comprises a light sensitive element and a signal output end connected to the light sensitive element. The light sensitive element is configured to receive the light via the light-transmitting unit and to generate an electrical signal based on the received light. The hand-held display unit is detachably coupled to the light-capturing unit to receive the electrical signal from the signal output end, and may comprise a hand-held portion and a display portion for displaying an image based on the received electrical signal.

Other objects, advantages and novel features of the present invention will be drawn from the following detailed embodiments of the present invention with attached drawings.

### BRIEF DESCRIPTION OF THE SEVERAL VIEWS OF THE DRAWINGS

The foregoing summary as well as the following detailed description of the preferred examples of the present invention will be better understood when read in conjunction with the appended drawings. For the purposes of illustrating the invention, there are shown in the drawings examples which are presently preferred. It is understood, however, that the invention is not limited to the precise arrangements and instrumentalities shown. In the drawings:

FIG. 1 illustrates schematic diagrams of various components of medical inspection devices in accordance with examples of the present invention.

FIG. 2 is a schematic diagram of an inspection unit of a medical inspection device in accordance with an example of the present invention.

FIG. 3 is a schematic diagram of a cross-section of a light-transmitting unit of a medical inspection device in accordance with an example of the present invention.

FIG. 4 is a schematic diagram of a cross-section of a light-transmitting unit of a medical inspection device in accordance with another example of the present invention.

FIG. 5 is a schematic diagram of a cross-section of a light-transmitting unit of a medical inspection device in accordance with yet another example of the present invention.

FIG. 6 is a schematic diagram of a cross-section of a light-capturing unit of a medical inspection device in accordance with an example of the present invention.

FIG. 7 is a schematic diagram of a cross-section of a light-capturing unit of a medical inspection device in accordance with another example of the present invention.

FIG. 8 is a schematic diagram of a hand-held display unit of a medical inspection device in accordance with an example of the present invention.

### DETAILED DESCRIPTION OF THE INVENTION

Reference will now be made in detail to the present examples of the invention illustrated in the accompanying drawings. Wherever possible, the same reference numbers will be used throughout the drawings to refer to the same or like portions. It should be noted that the drawings are made in simplified form and are not drawn to precise scale.

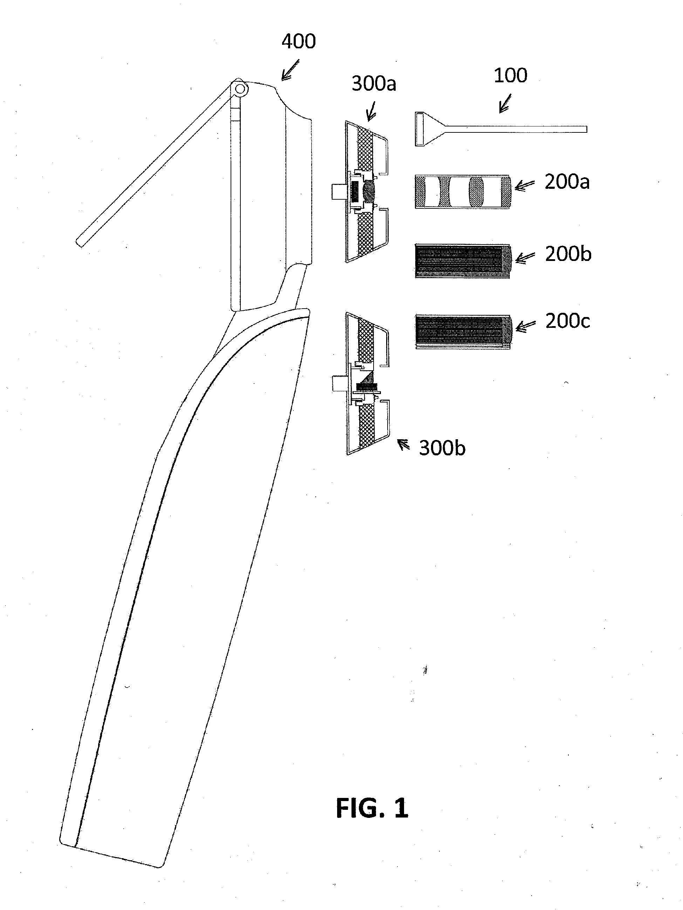

FIG. 1 illustrates schematic diagrams of various components of medical inspection devices in accordance with examples of the present invention. As illustrated in FIG. l, the medical inspection device may comprise an inspection unit 100 having a light-transmitting unit 200a, 200b or 200c disposed therein, a light-capturing unit 300a or 300b detachably coupled to the light-transmitting unit 200a, 200b or 200c and a hand-held display unit 400 detachably coupled to the light-capturing unit 300a or 300b.

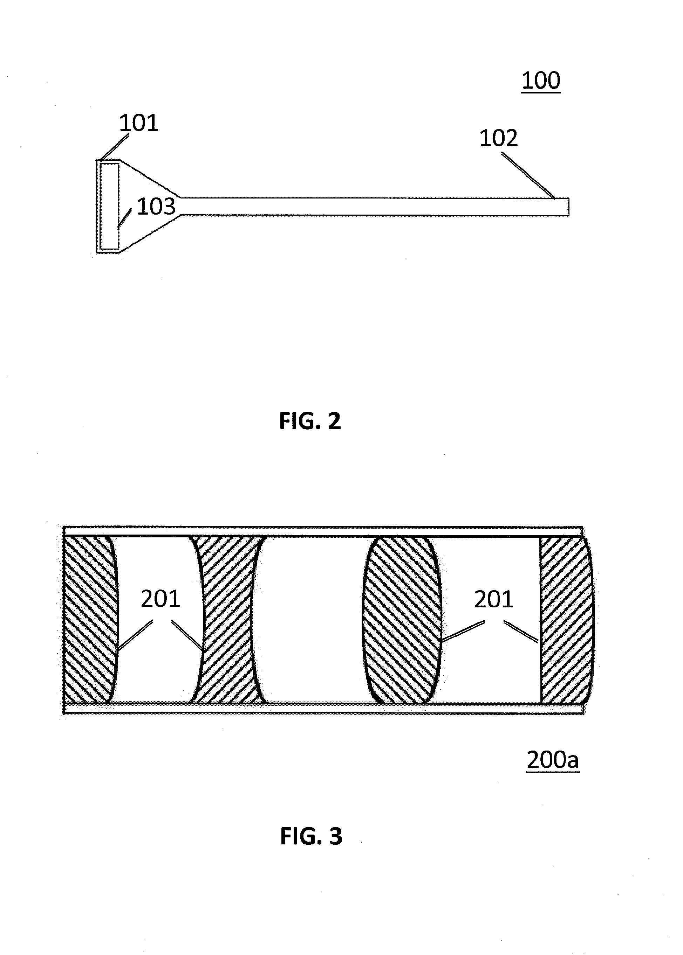

FIG. 2 is a schematic diagram of an inspection unit 100 of a medical inspection device in accordance with an example of the present invention. The inspection unit 100 as shown in FIG. 2 is a speculum for a rhinoscope. It will be appreciated by those skilled in the art that the shape of the inspection unit 100 is not limited to a conical shape. The inspection unit 100 may take any other shapes and forms designed for other medical inspection purposes, such as ophthalmascope, rhinoscope, dermatoscope, otoscope, arthroscope, endoscope, etc.

The inspection unit 100 comprises a front end 102, which has a light-transmitting unit 200a, 200b or 200c disposed therein. The inspection unit 100 further comprises a rear end 101. In accordance with an example of the present invention, the rear end 101 of the inspection unit 100 may include a coupling mechanism 103 which is configured to detachably couple the inspection unit 100 to a light-capturing unit 300a or 300b. Examples of the coupling mechanisms may include matching screw threads or latches.

According to other examples of the present invention, the medical inspection device may include the light transmitting unit 200a, 200b or 200c detachably coupled to the light-capturing unit 300a or 300b, and the hand-held display unit 400 detachably coupled to the light-capturing unit 300a or 300b as shown in Fig. 1. There are different ways of detachably coupling the light-transmitting unit 200a, 200b or 200c to the light-capturing unit 300a or 300b. A quick release clamping mechanism or fastening mechanism such as snap fit or latch may be provided to secure the light-capturing unit 300a or 300b to the light transmitting unit 200a, 200b or 200c. The present invention is not limited to specific coupling mechanisms as long as the light-transmitting unit 200a, 200b or 200c and light-capturing unit 300a or 300b have the mating structures to facilitate the coupling of the two units. In yet another example, a coupling mechanism having the mating structures for both the light-transmitting unit 200a, 200b or 200c and the light-capturing unit 300a or 300b may be provided in case the light transmitting unit 200a, 200b or 200c does not have the mating structures for the light capturing unit 300a or 300b.

FIG. 3 is a schematic diagram illustrating a cross-section of a light-transmitting unit 200a of a medical inspection device in accordance with an example of the present invention. Referring to FIG. 3, the light-transmitting unit 200a may comprise a set of optical lenses 201 for receiving and transmitting light. The set of optical lenses 201 may comprise an assembly of simple lenses or a combination thereof as a compound lens. In accordance with other examples of the invention, the set of optical lenses 201 may include a rod lens, cylinder lens or other similar optical elements. Furthermore, depending on the medical applications, one or more lenses of the set of optical lenses 201 may be polarized, colored, engraved with special patterns, filtered, coated or custom made in accordance with the user's need. The selection of the appropriate set of optical lenses 201 and their arrangement in the light-transmitting unit 200a may be determined appropriately by one skilled in the art.

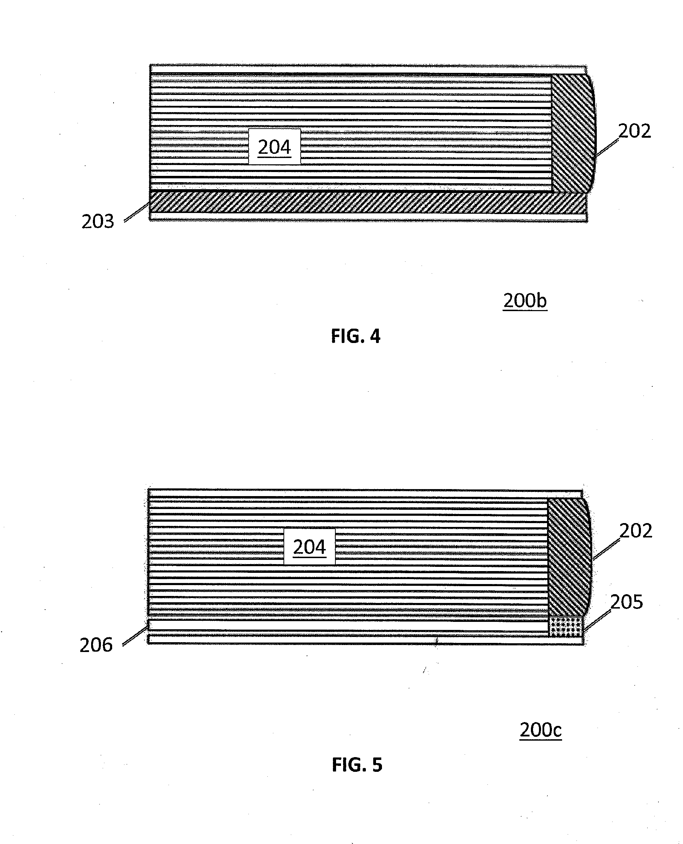

FIG. 4 is a schematic diagram illustrating a cross-section of a light-transmitting unit 200b of a medical inspection device in accordance with another example of the present invention. Referring to FIG. 4, the light-transmitting unit 200b comprises an optical lens 202 at the front end of the light-transmitting unit 200b for receiving external light and optical fibers 204 for transmitting the received light towards the rear end of the light-transmitting unit 200b. The light-transmitting unit 200b further comprises a light guide 203 for guiding light incident from an illuminating source in the light-capturing unit 300a or 300b or light reflected from an object.

FIG. 5 is a schematic diagram illustrating a cross-section of a light-transmitting unit 200c of a medical inspection device in accordance with yet another example of the present invention. The light-transmitting unit 200c may be similar to the light-transmitting unit 200b shown in FIG. 4 except that the light guide 203 of the light-transmitting unit 200b in FIG. 4 is replaced by a light source 205 disposed at the front end of the light-transmitting unit 200c and wiring 206 for connecting the light source 205 to the power. The light source 205 may include an electric powered light visible to the human eyes, for example, visible lasers, light emitting diodes (LEDs), other electroluminescent (EL) lamps, halogen lamp (incandescent lamps), gas discharged lamps, high intensity discharge lamps or electron stimulated lamps disposed at the periphery of the optical lens 202. In accordance with other examples of the present invention, the light source 205 may also be selected from a group consisting of other wavelengths of the electromagnetic spectrum not visible to human eyes. Some of the examples include radio waves, microwaves, InfraRed (IR), UltraViolet (UV), X rays, and gamma rays.

In addition, there may be other fittings at the front end 102 of the inspection unit 100 or the light-transmitting unit 200a, 200b or 200c according to other examples of the present invention. For example, rubber tubing that allows passage of micro operating tools may be provided to facilitate operation/treatment when the medical examination is carried out using the medical inspection device of the present invention. A speculum or protective sheath may also be provided to cover the inspection unit 100 or light-transmitting unit 200a, 200b or 200c partially or entirely to ensure a hygienic and safe use of the medical inspection device. In accordance with other examples of the invention, an eyecup or supporting piece may be included at the front end 102 of the inspection unit 100 or the light-transmitting unit 200a, 200b or 200c to ensure the eye is covered completely from surrounding light and positioned appropriately during the eye examination.

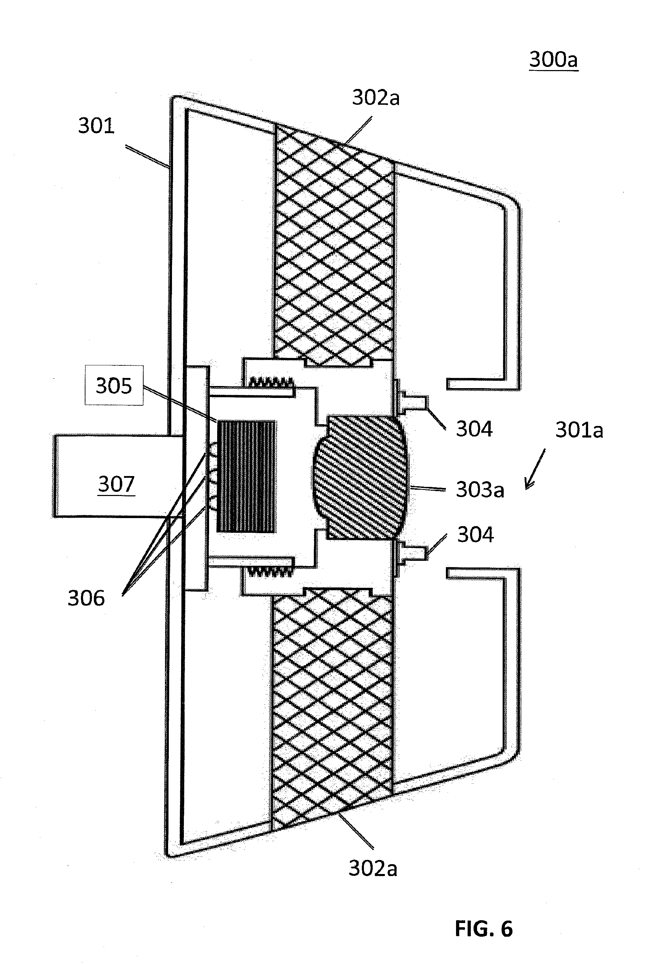

FIG. 6 is a schematic diagram illustrating a cross-section of a light-capturing unit 300a of a medical inspection device in accordance with an example of the present invention. As illustrated in FIG. 6, the light-capturing unit 300a may comprise a casing 301 containing a focusing lens 303a, which is held by a lens moving unit 302a. The casing 301 comprises and an opening 301a through which a light may enter the light-capturing unit 300a via the light transmitting unit 200a, 200b or 200c and be received by the focusing lens 303a. The lens moving unit 302a is configured to adjust the distance of the focusing lens 303a with respect to a light sensitive element 305 of the light-capturing unit 300a, so that the light received by the focusing lens 303a is projected onto the light sensitive element 305. The light sensitive element 305 is configured to receive the light via the light-transmitting unit 200a, 200b or 200c and to generate an electrical signal based on the received light. The light sensitive element 305 may be an image sensor such as a micro-sized (optical format: 1/4 inch or 1/3 inch) Complementary Metal Oxide Semiconductor (CMOS) sensor, a Charged Coupled Device (CCD) sensor, or an optical sensor, such as IR sensor, Radiofrequency (RF) sensor or sensors for detecting other light waves. According to a preferred embodiment, the light sensitive element 305 may be a video graphic array (VGA) image sensor, such as an OmniVision 7675 or OmniVision 7690 image sensor, or a quarter video graphics array (QVGA) image sensor, such as an OmniVision 6920 image sensor.

The light-capturing unit 300a may further comprise a signal output end 307, which receives electrical signals from the light sensitive element 305 via a plurality of electrode contacts 306 of the light sensitive element 305, and on another end, outputs the electrical signals to the hand-held display unit 400. The electrical signals are then converted into a visual signal which includes, but are not limited to, various video, image and graphical signals or figures to be displayed on the hand-held display unit 400. Examples of the video formats may include analog signals such as NTSC (National Television System Committee), PAL (Phase Alternating Lines), RGB (Red, Green, Blue) and other similar standards/interfaces, and digital signals such as RGB565, RGB666, RGB888, CCIR656 (Consultative Committee for International Radio 656), CCIR607, LVDS (Low Voltage Differential Signaling), MIPI (Mobile Industry Processor Interface) and other similar standards/interfaces. The signal output end 307 may be configured to output analog signal or be configured to output digital signal. In one example, the signal output end 307 is a Universal Serial Bus (USB) type connection including but not limited to micro USB, mini USB and USB connectors. In another example, the signal output end 307 comprises a plurality of electrical pads for sending signals of other formats.

In addition, the light-capturing unit 300a may further comprise one or more light sources 304 positioned around the focusing lens 303a. The light produced by the light sources 304 may also be directed to illuminate an object by a light guide 203 of the light transmitting unit 200b as shown in FIG. 4. Examples of the light sources 304 may include, but are not limited to, an electric powered light visible to the human eyes, for example, LEDs, other EL lamps, halogen lamp (incandescent lamps), gas discharged lamps, high intensity discharge lamps and electron stimulated lamps disposed at the periphery of the focusing lens 303a. In accordance with other examples of the present invention, the light sources 304 may also be selected from a group consisting of other wavelengths of the electromagnetic spectrum not visible to human eyes. Some of the examples include radio waves, microwaves, IR, UV, X rays, and gamma rays.

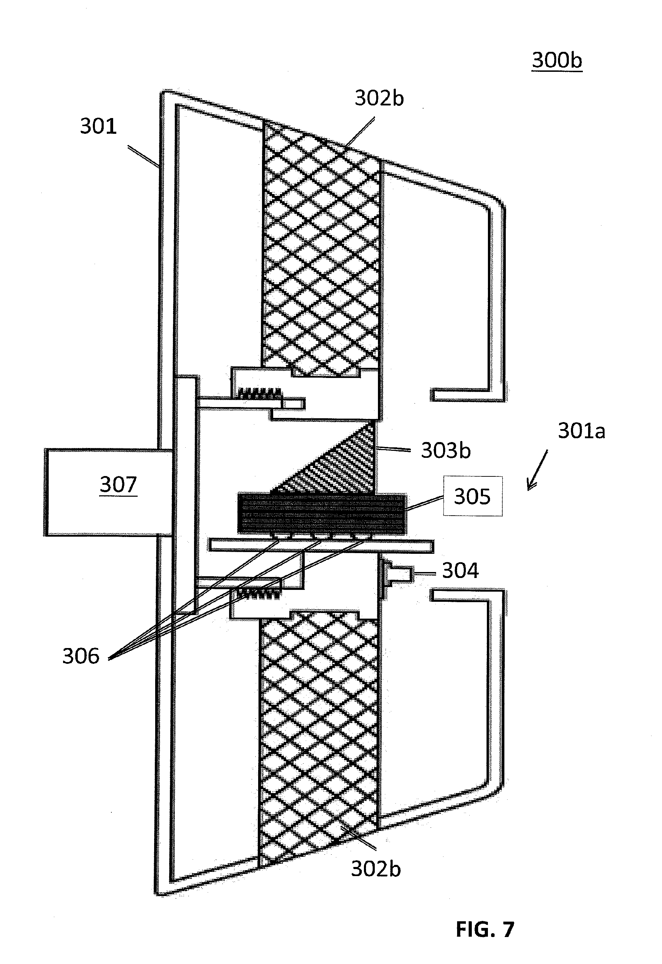

FIG. 7 is a schematic diagram illustrating a cross-section of a light-capturing unit 300b of a medical inspection device in accordance with another example of the present invention. The light-capturing unit 300b is similar to the light-capturing unit 300a shown in FIG. 6, except that the focusing lens 303a in FIG. 6 is replaced by a prism 303b, and the light sensitive element 305 is configured such that the surface of the light sensitive element 305 for receiving the light is perpendicular to the opening 301a of the casing 301. With such arrangement for the prism 303b and the light sensitive element 305, the size of the light-capturing unit 300b may be reduced dramatically to ensure a much more compact size for the medical inspection device. Furthermore, a lens moving unit 302b is configured to adjust the distance of both the prism 303b and the light sensitive element 305 with respect to the opening 301a. The prism may be coated with a reflective material to ensure that light enters from the opening 301a via the light transmitting unit 200a, 200b or 200c is reflected onto the light sensitive element 305 by the prism 303b. Again, an electrical signal is generated by the light sensitive element 305 based on the received light. The electrical signal, either processed or unprocessed, may be output via the signal output end 307 to the hand-held display unit 400.

Depending on the medical indications or applications desired, it may be possible to include in the light-capturing unit 300a or 300b other sensing devices or detectors for measuring and/or gauging the vital signs of the patients or test subjects. For example, there may be oxygen sensor, carbon dioxide sensor or temperature sensor arranged alongside or disposed adjacent to the light sensitive element 305 in the light-capturing unit 300a or 300b. There may even be ultrasonic sensor for measurement of the properties of sound waves with frequencies above the human audible range in sonography. Whenever a sound wave encounters a material with a different density, part of the sound wave is reflected back to the probe. The return of the sound wave vibrates the transducer, the transducer turns the vibrations into electrical pulses, which in turn are processed and transformed into a digital image. The sonography can be enhanced with Doppler measurements, which employ the Doppler effect to assess whether structures, usually blood, are moving towards or away from the probe, and its relative velocity. By calculating the frequency shift of a particular sample volume, for example, flow in an artery or a jet of blood flow over a heart valve, its speed and direction can be determined and visualized. This is particularly useful in cardiovascular studies.

For monitoring of cosmetic care, a medical inspection device such as a dermoscope or skin examining device may also include a moisture sensor for detecting the moisture level of the skin when the skin examination is conducted based on the features detected by other optical sensors. While it may be possible to include one or more of the above-mentioned sensors in the light-capturing unit 300a or 300b, it is noted that the final output signals displayed on the hand-held display unit may be superimposed, displayed side-by-side, and displayed sequentially to provide cross-reference of the medical examination results for the health professionals or the users of the medical inspection device to make a more precise and accurate diagnosis.

In another example of the present invention, the light-capturing unit 300a or 300b is a stand-alone unit which is configured to detachably couple to the inspection unit 100 on one end, and detachably couple to the hand-held display unit 400 on the other end.

According to other examples of the present invention, there are different ways of detachably coupling the light-capturing unit 300a or 300b to the hand-held display unit 400. Besides an annular protuberance settled on one surface of the hand-held display unit 400, a hinged anchor may be formed on one edge of the hand-held display unit 400, preferably on the distal surface, to jointly fit with a corresponding joint pin formed on the rear end of the light-capturing unit 300a or 300b. A fastening mechanism, such as snap fit or latch, may be provided to secure the light-capturing unit 300a or 300b to the hand-held display unit 400. The hand-held display unit 400 may also be equipped with a slider track mechanism to ensure easy detachment or assembly of the light-capturing unit from/to the hand-held display unit 400.

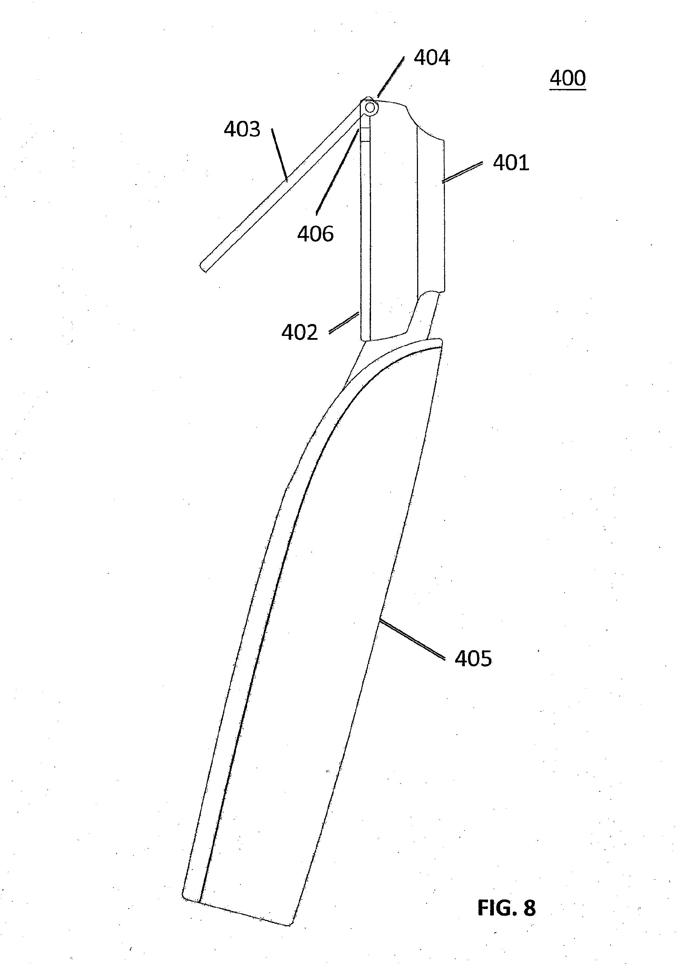

FIG. 8 is a schematic diagram of the hand-held display unit 400 of a medical inspection device in accordance with an example of the present invention. As illustrated in FIG. 8, the hand-held display unit 400 may comprise an engagement portion 401 and a display device 402. The engagement portion 401 is configured to detachably couple with the light-capturing unit 300a or 300b or the inspection unit 100. Furthermore, the engagement portion 401 may comprise a receptacle which may engage with and be electrically connected to the signal output end 307. The receptacle may be a USB connection or comprise a plurality of electrical pads. The hand-held display unit 400 may further comprise a display cover 403 and a hinge 404 for attaching the display cover 403 to the hand-held display unit 400. Moreover, the medical inspection device may further comprise a hand-held portion 405 and an image projecting unit 406 adjacent to the display device 402. It will be appreciated by those skilled in the art that the display device 402 of the present invention may include, but is not limited to, Thin Film Transistor Liquid Crystal Display (TFT LCD), LED, Active Matrix Organic Light Emitting Diode (AMOLED) and other backlit displays including or excluding the resistive or capacitive touchscreen functions, with the dimension ranging from approximately 1 inch to 13 inches in diagonal length. It is also understood by those skilled in the art that the display device 402 may be designed to include various user interfaces, data manipulation and system operating functions desired. In addition, the medical inspection device may further comprise a data storage medium (not shown) for storing images captured by the medical inspection device. Alternatively, the hand-held display unit 400 may be further configured for coupling to a peripheral storage device for storing images or videos captured by the medical inspection device. To ensure the signal output to the display device 402 is shared and communicated with other systems having a display function, a signal transmitting unit, either wired or wireless may also be included and built on the hand-held display unit 400.

It will be appreciated by those skilled in the art that the hand-held display unit 400 of the present invention is not limited to those illustrated in the drawings. Other types of the hand-held display unit with different configurations may also be encompassed within the scope of the present invention. For example, other supporting structures, such as strap, belt, buckle, hooks, pods, etc., may be provided on the hand-held display unit to increase its stability and portability when it is not held by the user. It is also understood by those skilled in the art that the medical inspection device of the present invention may also be coupled via the hand-held display unit 400 to other peripheral devices, such as external display device, storage device, charging station, cleaning station, wireless signal transmitter, etc., to expand the applications of the medical inspection device.

The medical inspection device in accordance with the present invention further comprises a signal processor, such as encoder or decoder, or a signal converter, such as analog-to-digital (A/D) converters or digital-to-analog (D/A) converters (not shown), which is electrically coupled to the light sensitive element 305 for processing signals received from the light sensitive element 305. The signal processor may be disposed within the light-capturing unit 300a or 300b or within the hand-held display unit 400 depending on the actual design of the medical inspection device.

The present invention provides the options of selecting light sensitive elements which match with the desired optical elements and/or light source depending on the application of the medical inspection device. The user may select the appropriate light-capturing unit 300a or 300b, light-transmitting unit 200a, 200b or 200c, and hand-held display unit 400 in accordance with their needs and budget. In addition, sensor upgrade will be more affordable since the light-capturing unit 300a or 300b, which comprises the light sensitive element 305, is not made as an integral part of the hand-held display unit 400 or a part of the light-transmitting unit 200a, 200b or 200c. On the other hand, the user has the option of choosing the desired optical element in the light-transmitting unit 200a, 200b or 200c to match with the light sensitive element 305 according to the medical examination carried out by the user. Since it is possible to freely detach the light-transmitting unit 200a, 200b or 200c from the light-capturing unit 300a or 300b and to detach the light-capturing unit 300a or 300b from the hand-held display unit 400, it is also more affordable to replace each module or unit in the entire package of the medical inspection device which encompasses a free combination of the light-transmitting unit 200a, 200b or 200c, light-capturing unit 300a or 300b and hand-held display unit 400 according to the present invention.

It will be appreciated by those skilled in the art that changes could be made to the examples described above without departing from the broad inventive concept thereof. It is understood, therefore, that this invention is not limited to the particular examples disclosed, but it is intended to cover modifications within the spirit and scope of the present invention as defined by the appended claims.

## Claims

1. A medical inspection device comprising:
a light-transmitting unit comprising at least a first optical lens for receiving and transmitting light;
a light-capturing unit detachably coupled to the light-transmitting unit, the light-capturing unit comprising a light sensitive element and a signal output end connected to the light sensitive element, wherein the light sensitive element is configured to receive the light via the light-transmitting unit and to generate an electrical signal based on the received light; and
a hand-held display unit detachably coupled to the light-capturing unit to receive the electrical signal from the signal output end, the hand-held display unit comprising a hand-held portion and a display portion for displaying a visual signal based on the received electrical signal.

2. The medical inspection device according to claim 1, wherein the light-transmitting unit further comprises a light guide for directing light from the light source to an object.

3. The medical inspection device according to claim 1, wherein the hand-held display unit further comprises a receptacle for electrically connecting to the signal output end.

4. The medical inspection device according to any one of claims 1 to 3, wherein the signal output end includes a USB connector.

5. The medical inspection device according to claim 1, wherein the hand-held display unit further comprises a data storage medium for storing the image captured by the light capturing unit.

6. The medical inspection device according to claim 1 further comprises a coupling device for connecting the light transmitting unit to the light capturing unit.

7. The medical inspection device according to claim 1, further comprising:
an inspection unit having a front end with the light-transmitting unit disposed therein and a rear end, wherein the light-capturing unit is detachably coupled to the rear end of the inspection unit.

8. The medical inspection device according to claim 7 further comprising a coupling mechanism at the rear end of the inspection unit.

9. The medical inspection device according to claim 1 or 7, wherein the light-capturing unit further comprises a lens moving unit holding a second optical lens over the light sensitive element, wherein the lens moving unit is configured to adjust the distance of the second optical lens with respect to the light sensitive element.

10. The medical inspection device according to claim 1 or 7, wherein the light-capturing unit further comprises a lens moving unit holding a prism and the light sensitive element, wherein the lens moving unit is configured to adjust the distance of the prism and the light sensitive element with respect to an opening of the light-capturing unit.

11. The medical inspection device according to claim 1 or 7, wherein the light-transmitting unit further comprises a light source peripheral to the first optical lens.

12. The medical inspection device according to claim 1 or 7, wherein the light-capturing unit further comprises a light source peripheral to the light sensitive element.

13. The medical inspection device according to claim 7, wherein the light-transmitting unit further comprises a light guide for directing light from an illumination source to an object.

14. The medical inspection device according to claim 1 or 7, wherein the hand-held display unit further comprises an image projecting device adjacent to the display portion.

15. The medical inspection device according to claim 7, wherein the hand-held display unit further comprises a data storage medium for storing the image captured by the light-capturing unit.
